# EUROPEAN PATENT APPLICATION

(11) **EP 3 964 153 A1**
(43) Date of publication of application: **09.03.2022**
(21) Application number: 21165832.3
(22) Date of filing: 30.03.2021
(51) Int. Cl.: A61B 18/14

(54) **IMPEDANCE BASED IRREVERSIBLE-ELECTROPORATION (IRE)**

(30) Priority: 08.09.2020 US 202017014221
(71) Applicant: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(57) **Abstract**

An irreversible electroporation (IRE) method includes selecting electrodes of a catheter placed in contact with tissue in an organ, for applying IRE pulses between the selected electrodes. An impedance is measured between the selected electrodes. Based on the measured impedance, an IRE protocol is chosen, that has parameters that meet a predefined safety criterion under the measured impedance. The IRE pulses are applied according to the chosen protocol.

## Description

### FIELD OF THE INVENTION

The present invention relates generally to invasive ablation, and particularly to irreversible electroporation (IRE) of cardiac tissue.

### BACKGROUND OF THE INVENTION

Estimation of invasive ablation parameters and controlling the ablation according to the estimation has been previously proposed in the patent literature. For example, U.S. Patent Application Publication No. 2013/0006228 describes devices for localized delivery of energy and methods of using such devices, particularly for therapeutic treatment of biological tissues. The disclosed methods may involve positioning and deploying the energy delivery members in a target site, and delivering energy through the energy delivery members. In an embodiment, radiofrequency (RF) duty cycle and/or pulse duration can be configured to vary in response to one or more selected parameters, which can include frequency of the treatment signal, power for the treatment signal, or tissue impedance to the treatment signal.

As another example, U.S. Patent Application Publication No. 2016/0066977 describes a medical system for ablating a tissue site with real-time monitoring during an electroporation treatment procedure. A pulse generator generates a pre-treatment test signal having a frequency of at least 1 MHz prior to the treatment procedure and intra-treatment test signals during the treatment procedure. A treatment control module determines impedance values from the pre-treatment test signal and intra-treatment test signals and determines a progress of electroporation and an end point of treatment in real-time based on the determined impedance values while the treatment progresses.

### SUMMARY OF THE INVENTION

An embodiment of the present invention that is described herein after provides an irreversible electroporation (IRE) method including selecting electrodes of a catheter placed in contact with tissue in an organ, for applying IRE pulses between the selected electrodes. An impedance is measured between the selected electrodes. Based on the measured impedance, an IRE protocol is chosen, that has parameters that meet a predefined safety criterion under the measured impedance. The IRE pulses are applied according to the chosen protocol.

In some embodiments, choosing the IRE protocol includes choosing a pulse width, a pulse repetition rate and a number of the IRE pulses.

In some embodiments, choosing the predefined safety criterion includes choosing an IRE protocol having an impedance threshold that is lower than the measured impedance, thereby ensuring that the IRE pulses will not overheat the tissue.

In an embodiment, the impedance threshold of the chosen protocol is derived by one of a calculation and a look up table.

In another embodiment, the IRE method further includes setting an IRE protocol including the impedance threshold. The impedance is measured between the selected electrodes and the measured impedance is compared to the given threshold. If the measured impedance is below the threshold, the IRE protocol is changed to have a new impedance threshold that is below the measured impedance. The IRE is applied according to the changed protocol.

In an embodiment, setting the IRE protocol includes selecting a pulse width, a pulse repetition rate and a number of IRE pulses, and changing the IRE protocol includes changing one or more of the pulse width, the pulse repetition rate and the number of IRE pulses. In another embodiment, changing the one or more of the pulse width, the pulse repetition rate and the number of IRE pulses includes reducing one of the pulse width and the repetition rate, and increasing the number of pulses.

In some embodiments, changing the IRE protocol includes maintaining a same accumulated electrical energy applied by the IRE.

There is additionally provided, in accordance with another embodiment of the present invention, an irreversible electroporation (IRE) system including an interface and a processor. The interface is configured to exchange signals with a catheter placed in contact with tissue in an organ. The processor is configured to (i) select electrodes of the catheter for applying IRE pulses between the selected electrodes, (ii) measure an impedance between the selected electrodes, (iii) choose, based on the measured impedance, an IRE protocol having parameters that meet a predefined safety criterion under the measured impedance, and (iv) apply the IRE pulses according to the chosen protocol.

### BRIEF DESCRIPTION OF THE DRAWINGS

The present invention will be more fully understood from the following detailed description of the embodiments thereof, taken together with the drawings in which:
Fig. 1 is a schematic, pictorial illustration of a catheter-based irreversible electroporation (IRE) system, in accordance with an exemplary embodiment of the present invention; and
Fig. 2 is a flow chart that schematically illustrates a method for applying irreversible electroporation (IRE) pulses using the system of Fig. 1, in accordance with an exemplary embodiment of the present invention.

### DETAILED DESCRIPTION OF EMBODIMENTS

### OVERVIEW

Irreversible electroporation (IRE), also called Pulsed Field Ablation (PFA), may be used as an invasive therapeutic modality to kill tissue cells by subjecting them to high-voltage pulses. Specifically, IRE pulses have a potential use to kill myocardium tissue cells in order to treat cardiac arrhythmia. Cellular destruction occurs when the transmembrane potential exceeds a threshold, leading to cell death and thus the development of a tissue lesion. Therefore, of particular interest is the use of high-voltage bipolar electric pulses (e.g., using a selected pair of electrodes in contact with tissue) to generate high electric fields (e.g., above a certain threshold) to kill tissue cells between the electrodes.

However, if the electrical impedance between the selected electrodes is low, then the high voltage between the electrodes may also generate excessive Joule heating that might cause unwanted effects of potential clinical hazard, such as steam popping in the ablated tissue. For example, a pulse voltage of 2 kV across 100 Ω (both possible values) momentarily generates 20 Amps, i.e., 40 kW in the tissue, which may be hazardous.

Embodiments of the present invention that are described hereinafter protect from excessive Joule heating of tissue undergoing IRE, by taking into account the electrical impedance between electrodes. In one embodiment, initially, an IRE ablation protocol, such as a default protocol, is selected, with this protocol typically specifying the pulse shape (peak voltage and width), pulse repetition rate, and number of pulses.

The selected protocol can be viewed as having a certain impedance threshold, i.e., a threshold that the actual electrical impedance between the electrodes must exceed in order for the ablation to be considered safe. The impedance threshold for a given protocol may be, for example, calculated by a processor based on the protocol parameters, or read from a predetermined relation (e.g., from an empirically-derived or pre-calculated relation that is stored in a look up table).

Some embodiments use the electrodes selected for the IRE to measure the impedance between the electrodes. If the measured impedance is above the impedance threshold, meaning Joule heating is within acceptable range, then the processor commands the system to perform the IRE using the initial protocol.

If, however, the measured impedance is below the impedance threshold, in which case the selected protocol may cause excessive heating, then the processor, or a user, changes the initial protocol. The change may involve shortening individual pulse length times, decreasing the repetition rate of pulses, and/or increasing the number of pulses (to maintain a same cumulative clinical effect). The changed protocol has a different impedance threshold (e.g., a lower threshold), so that the same measured impedance is now above the new threshold, and IRE can be performed.

Ablative-energy wise, in order to ablate at the given impedance, if the impedance is below the threshold, the protocol is adjusted to maintain as much as possible a similar energy delivery. The optimization is to modify the pulse duration and/or number of pulses and/or number of bursts to maintain similar energy delivery to get the same clinical and thermal effect. For ablation with lower impedance, total energy is nevertheless reduced by modifying pulse duration and/or number of pulses and the impedance threshold is adjusted accordingly, to a maximal allowable value.

When changing the protocol, the peak voltage of the IRE pulses is typically not reduced, since this affects the electroporation field generated. In some embodiments the peak voltage may be reduced, as long as it is kept above a predefined minimum level required for the IRE to be clinically effective.

As noted above, to maintain clinical effect, the changes to the protocol typically do not change the overall (e.g., accumulated) energy dissipated. Rather, the changes spread out the time of application of the pulses, so as to permit heat generated to diffuse more and lower a maximum temperature caused by the heating.

In another embodiment, the physician first inserts and places the catheter at a target tissue location. Next, the IRE ablation system measures an impedance between the electrodes of the catheter. Then, the system or the physician choose, based on the measured impedance, an IRE protocol having parameters that meet a predefined safety criterion under the measured impedances. Any suitable safety criterion can be used. For example, a safety criterion aiming at minimizing a risk of over-heating tissue is provided, that is based on a minimal impedance measured, where the system chooses a protocol having an impedance threshold that is lower than the minimal measured impedance, so that a resulting electrical current will be below one causing over-heating of tissue. Such a protocol can therefore be used safely, as the expected Joule heating is within an acceptable range. In this embodiment, there is no need to initially select a protocol (e.g., to upload a protocol and to subsequently modify it based on a measured impedance).

The disclosed technique can be used in various types of IRE ablation procedures and with various types of catheters. In one example, cardiac IRE ablation is performed using an expandable frame (e.g., balloon or basket) fitted on a distal end of an ablation catheter. The expandable frame, which is disposed with ablation electrodes, is navigated through the cardiovascular system and inserted into a heart to, for example, ablate an ostium of a pulmonary vein (PV).

By making the above described changes to the IRE protocol, IRE ablation of, for example an ostium of a PV using an expandable frame catheter, can be made safer, while maintaining its clinical efficacy.

### SYSTEM DESCRIPTION

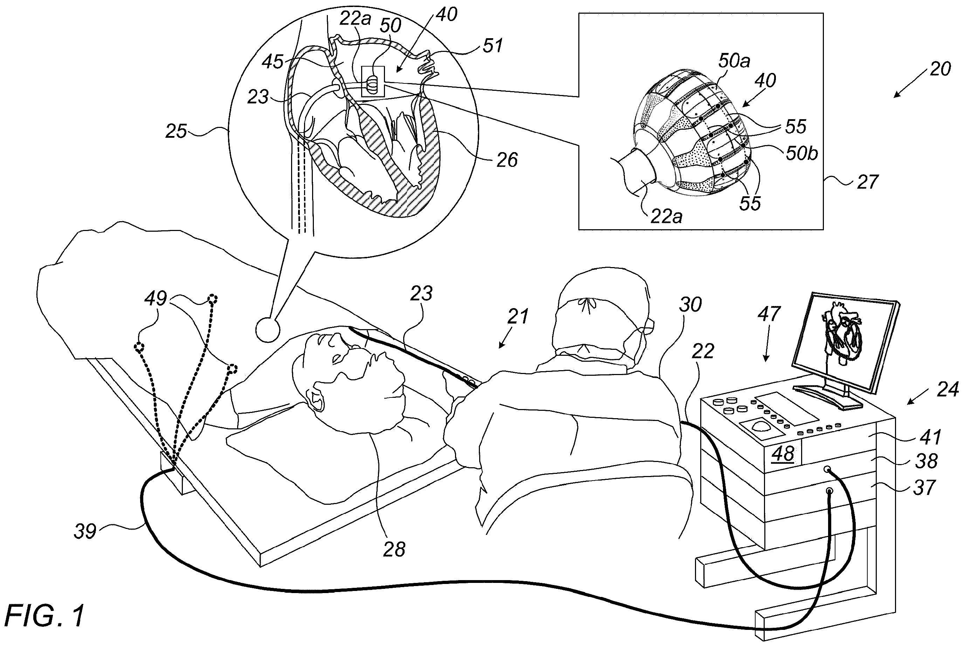

Fig. 1 is a schematic, pictorial illustration of a catheter-based irreversible electroporation (IRE) system 20, in accordance with an embodiment of the present invention. System 20 comprises a catheter 21, wherein a shaft 22 of the catheter is inserted by a physician 30 through the vascular system of a patient 28 through a sheath 23. The physician 30 then navigates a distal end 22a of shaft 22 to a target location inside a heart 26 of the patient (inset 25).

Once distal end 22a of shaft 22 has reached the target location, physician 30 retracts sheath 23 and expands balloon 40, typically by pumping saline into balloon 40. Physician 30 then manipulates shaft 22 such that electrodes 50 disposed on balloon catheter 40 engage an interior wall of a PV ostium 51 to apply high-voltage IRE pulses via electrodes 50 to ostium 51 tissue.

As seen in insets 25 and 27, distal end 22a is fitted an expandable balloon 40 comprising multiple smooth-edge and equidistant IRE electrodes 50. Due to the flattened shape of the distal portion of balloon 40, distances between adjacent electrodes 50, such as a distance 55 between electrodes 50a and 50b, remains approximately constant even where electrodes 50 cover the distal portion. Balloon 40 configuration therefore allows more effective (e.g., with approximately uniform electric field strength) electroporation between adjacent electrodes 50 while the smooth edges of electrodes 50 minimize unwanted thermal effects.

Certain aspects of inflatable balloons are addressed, for example, in U.S. Provisional Patent Application No. 62/899,259, filed September 12, 2019, titled "Balloon Catheter with Force Sensor," and in U.S. Patent Application No. 16/726,605, filed December 24, 2019, titled, "Contact Force Spring with Mechanical Stops," which are both assigned to the assignee of the present patent application and whose disclosures are incorporated herein by reference.

In the embodiment described herein, catheter 21 may be used for any suitable diagnostic purpose and/or therapeutic purpose, such as electrophysiological sensing and/or the aforementioned IRE isolation of PV ostium 51 tissue in left atrium 45 of heart 26.

The proximal end of catheter 21 is connected to a console 24 comprising an IRE pulse generator 38 configured to apply the IRE pulses between adjacent electrodes 50. The electrodes are connected to IRE pulse generator 38 by electrical wiring running in shaft 22 of catheter 21. A memory 48 of console 24 stores IRE protocols comprising IRE pulse parameters, such as peak bi-polar voltage and pulse width are stored.

Console 24 comprises a processor 41, typically a general-purpose computer, with suitable front end and interface circuits 37 for receiving signals from catheter 21 and from external electrodes 49, which are typically placed around the chest of patient 26. For this purpose, processor 41 is connected to external electrodes 49 by wires running through a cable 39.

During a procedure, system 20 can track the respective locations of electrodes 50 inside heart 26, using the Active Current Location (ACL) method, provided by Biosense-Webster (Irvine California), which is described in US Patent No. 8,456,182, whose disclosure is incorporated herein by reference.

In some embodiments, interface circuits 37 are configured to measure an impedance between selected electrodes among electrodes 50, such as an impedance between electrodes 50a and 50b, after balloon 40 is placed at target tissue but before an IRE is performed, and provide the measured impedance to processor 41. The processor compares the impedance to a predetermined impedance threshold, and if the impedance are below the threshold, processor 41, or alerted physician 30 (by the system), changes the protocol as described, for example, in tables I and II below, so as to mitigate thermal hazard while maintaining clinical efficacy of the IRE.

In other embodiments, physician 30 can modify, from a user-interface 47, any of the parameters of the changed protocol.

Processor 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

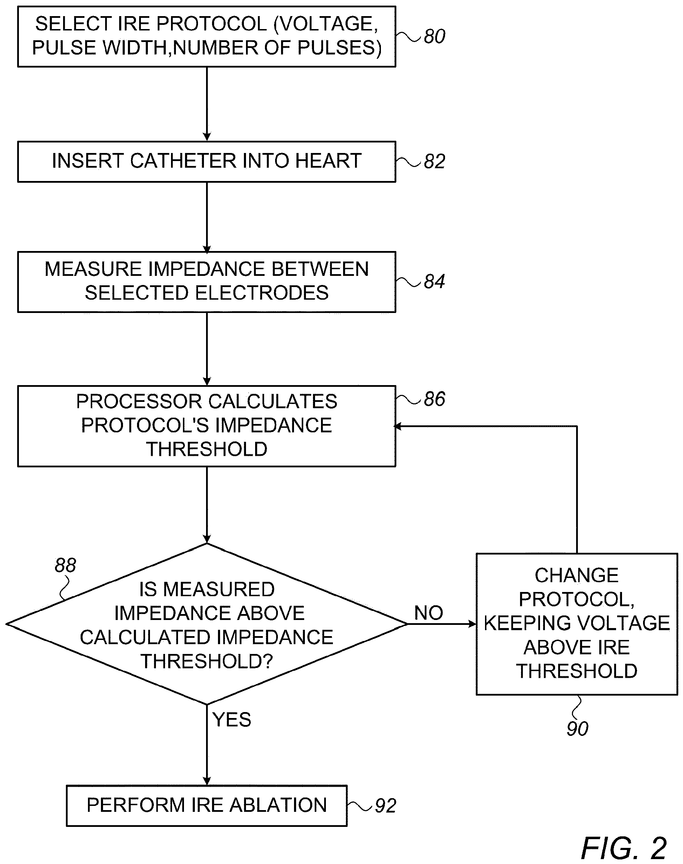

In particular, processor 41 runs a dedicated algorithm as disclosed herein, including in Fig. 2, that enables processor 41 to perform the disclosed steps, as further described below. In particular, processor 41 is configured to command IRE pulse generator 38 to output IRE pulses according to a treatment protocol that processor 41 uploads from memory 48.

### METHOD OF IMPEDANCE BASED IRE

Fig. 2 is a flow chart that schematically illustrates a method for applying irreversible electroporation (IRE) pulses using system 20 of Fig. 1, in accordance with an embodiment of the present invention. The algorithm, according to the presented embodiment, carries out a process that begins at an IRE planning step 80, when the physician uses processor 41 to upload a protocol with parameters of the IRE pulses to apply to in tissue. An example of IRE ablation settings in an initial protocol that may be used for ablating cardiac tissue using the disclosed balloon 40 is given in table I:

**Table I - Initial protocol**

| **Parameter** | **Value** |
|---|---|
| Preset IRE peak voltage | 1000 V |
| Pulse width | 1 mSec |
| Repetition rate | 2 Hz |
| Number of pulses | 10 |

Next, physician 30 inserts and navigates balloon 40 catheter to a target tissue location in an organ of a patient, such as at PV ostium 51, using, for example, electrode 50 as ACL sensing electrodes, at a balloon catheter navigation step 82.

Next, at an impedance measurement step 84, processor 41 measures an impedance between selected electrodes (e.g., using interface circuits 37).

At an impedance threshold determination step 86, processor 41 outputs (e.g., calculated or selects from a look up table) the uploaded protocol's impedance threshold.

At an impedance checking step 88, processor 41 compares the measured impedance to the impedance threshold. If the measured impedance is below the protocol's impedance threshold, the physician uses processor 41 to, in a protocol change step 90, change the protocol to that given, for example, in table II:

**Table II - Changed protocol**

| **Parameter** | **Range** |
|---|---|
| Preset IRE peak voltage | 1000 V |
| Pulse width | 0.5 mSec |
| Repetition rate | 1 Hz |
| Number of pulses | 40 |

As seen in table II, while pulse width and repetition rate decrease, the accumulated electrical energy is kept a same by increasing the number of pulses.

In other words, given the measured impedance, the protocol's parameters, except the voltage, are modified so the energy will be kept similar to the planned value. The impedance threshold is adjusted below the measured impedance because it was optimized in this manner. As a result, the impedance threshold value, automatically adjusted for the changed protocol (e.g., that of Table II), moves below the same measured impedance, then, using the changed IRE pulse parameters, processor 41 commands generator 38 to apply the IRE pulses to tissue, at an IRE treatment step 92. The IRE pulses are applied between selected electrodes of balloon 40 to isolate an arrhythmia originating or propagating via ostium 51.

If, on the other hand, the changed protocol yields a new impedance threshold still above the measured impedance, the process repeats by going back to step 90 and further changing the IRE protocol.

Although the embodiments described herein mainly address cardiac applications, the methods and systems described herein can also be used in other medical applications, such as in ablation of liver or lung cancers, and in neurology and otolaryngology.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

### ASPECTS OF THE INVENTION

1. An irreversible electroporation (IRE) method, comprising:
   selecting electrodes of a catheter placed in contact with tissue in an organ, for applying IRE pulses between the selected electrodes;
   measuring an impedance between the selected electrodes;
   choosing, based on the measured impedance, an IRE protocol having parameters that meet a predefined safety criterion under the measured impedance; and
   applying the IRE pulses according to the chosen protocol.
2. The IRE method according to aspect 1, wherein choosing the IRE protocol comprises choosing a pulse width, a pulse repetition rate and a number of the IRE pulses.
3. The IRE method according to aspect 1, wherein choosing the predefined safety criterion comprises choosing an IRE protocol having an impedance threshold that is lower than the measured impedance, thereby ensuring that the IRE pulses will not overheat the tissue.
4. The IRE method according to aspect 3, wherein the impedance threshold of the chosen protocol is derived by one of a calculation and a look up table.
5. The IRE method according to aspect 3, and comprising:
   setting an IRE protocol comprising the impedance threshold;
   measuring the impedance between the selected electrodes and comparing the measured impedance to the given threshold;
   if the measured impedance is below the threshold, changing the IRE protocol to have a new impedance threshold that is below the measured impedance; and
   applying the IRE according to the changed protocol.
6. The IRE method according to aspect 5, wherein setting the IRE protocol comprises selecting a pulse width, a pulse repetition rate and a number of IRE pulses, and wherein changing the IRE protocol comprises changing one or more of the pulse width, the pulse repetition rate and the number of IRE pulses.
7. The IRE method according to aspect 6, wherein changing the one or more of the pulse width, the pulse repetition rate and the number of IRE pulses comprises reducing one of the pulse width and the repetition rate, and increasing the number of pulses.
8. The method according to aspect 5, wherein changing the IRE protocol comprises maintaining a same accumulated electrical energy applied by the IRE.

## Claims

1. An irreversible electroporation (IRE) system, comprising:
an interface configured to exchange signals with a catheter placed in contact with tissue in an organ; and
a processor, which is configured to:
select electrodes of the catheter for applying IRE pulses between the selected electrodes;
measure an impedance between the selected electrodes;
choose, based on the measured impedance, an IRE protocol having parameters that meet a predefined safety criterion under the measured impedance; and
apply the IRE pulses according to the chosen protocol.

2. The IRE system according to claim 1, wherein the processor is configured to choose the IRE protocol by choosing a pulse width, a pulse repetition rate and a number of the IRE pulses.

3. The IRE system according to claim 1, wherein the processor is configured to choose the predefined safety criterion by choosing an IRE protocol having an impedance threshold that is lower than the measured impedance, thereby ensuring that the IRE pulses will not overheat the tissue.

4. The IRE system according to claim 3, wherein the processor is configured to derive the impedance threshold of the chosen protocol by one of a calculation and a look up table.

5. The IRE system according to claim 3, wherein the processor is configured to:
set an IRE protocol comprising the impedance threshold;
measure the impedance between the selected electrodes;
compare the measured impedance to the given threshold;
if the measured impedance is below the threshold, change the IRE protocol to have a new impedance threshold that is below the measured impedance; and
apply the IRE according to the changed protocol.

6. The system according to claim 5, wherein the processor is configured to set the IRE protocol by selecting a pulse width, a pulse repetition rate and a number of the IRE pulses, and to change the IRE protocol by changing one or more of the pulse width, the pulse repetition rate and the number of IRE pulses.

7. The system according to claim 6, wherein the processor is configured to reduce one of the pulse width and repetition rate, and to increase the number of pulses.

8. The system according to claim 5, wherein the processor is configured to change the IRE protocol while maintaining a same accumulated electrical energy applied by the IRE.
